# EUROPÄISCHE PATENTANMELDUNG

(11) **EP 1 829 974 A1**
(43) Veröffentlichungstag der Anmeldung: **05.09.2007**
(21) Anmeldenummer: 07102435.0
(22) Anmeldetag: 15.02.2007
(51) Int. Cl.: C12P 7/16, C12P 41/00

(54) **Verfahren zur Herstellung von (S)-2-Butanol und 2-Butanon aus racemischem 2-Butanol unter Verwendung einer Alkoholdehydrogenase**

(30) Priorität: 02.03.2006 DE 102006009744
(71) Anmelder: Wacker Chemie AG, 81737 München (DE)
(72) Erfinder: Pfaller, Rupert, 80639, München (DE); Schneider, Christina, 83104, Hohenthann (DE)
(74) Vertreter: Potten, Holger

(57) **Zusammenfassung**

Die Erfindung betrifft ein Verfahren, zur Herstellung von (S)-2-Butanol einer optischen Reinheit ee von > 90 %, bei dem eine Biotransformationszusammensetzung, umfassend als Edukt ein racemisches Gemisch von 2-Butanol (rac-2-Butanol), eine Oxidoreduktase, einen Redox-Cofaktor und ein Cosubstrat zur Reaktion gebracht wird, wobei selektiv (R)-2-Butanol zu 2-Butanon oxidiert wird und (S)-2-Butanol in einer optischen Reinheit ee von > 90 % entsteht.

## Beschreibung

Die Erfindung betrifft ein Verfahren zur Herstellung von (S)-2-Butanol durch oxidative enzymatische Spaltung eines racemischen Gemisches von 2-Butanol (rac-2-Butanol).

Optisch aktive Hydroxyverbindungen sind wertvolle Synthesebausteine, z. B. bei der Herstellung pharmazeutischer Wirkstoffe oder von Agrochemikalien. Sie haben daher eine wichtige wirtschaftliche Bedeutung. Diese Verbindungen sind durch klassische chemische Verfahren oft nur schwer herstellbar. Die erforderlichen optischen Reinheiten für Anwendungen im pharmazeutischen oder agrochemischen Bereich sind auf diesem Wege nur schwer zu erreichen. Daher werden zur Herstellung chiraler Verbindungen im zunehmenden Maße biotechnologische Verfahren angewendet. Speziell Enzyme, die Carbonylverbindungen reduzieren können bzw. Alkohole selektiv oxidieren können, finden wegen ihrer hohen Enantioselektivität vermehrt Verwendung.

Enzyme aus der Klasse der Oxidoreduktasen, die zur Herstellung chiraler Verbindungen durch Reduktion prochiraler Carbonylverbindungen eingesetzt werden, werden definitionsgemäß mit dem Sammelbegriff Carbonylreduktasen (in der Folge "CR") bezeichnet. In der Mehrzahl der Fälle ist das Produkt einer CR-Reaktion ein Alkohol. Es ist aber auch möglich, dass das Produkt einer CR-Reaktion ein Amin ist. Zu den unter dem Sammelbegriff Carbonylreduktasen zusammengefassten Enzymklassen zählen unter anderen Alkohldehydrogenasen (in der Folge "ADH"), Aldo-Ketoreduktasen, Aldehydreduktasen, Glycerindehydrogenasen und die Fettsäuresynthase. Diesem breiten Spektrum reduzierender Enzyme ist gemeinsam, dass sie die Elektronen für die Reduktion der Carbonylverbindung aus Redox-Cofaktoren in deren reduzierter Form, üblicherweise NADH oder NADPH, beziehen.

CRs aus der Klasse der ADHs sind zu reversiblen Redoxreaktionen befähigt, d. h. bestimmte prochirale Carbonylverbindungen werden nicht nur zum Alkohol reduziert, sondern der Alkohol kann auch zur Carbonylverbindung oxidiert werden. Wegen der hohen Enantioselektivität mancher ADHs kann dies für die oxidative Racematspaltung ausgenützt werden.

Die Redox-Cofaktoren NAD bzw. NADP werden bei der oxidativen Racematspaltung stöchiometrisch verbraucht, d.h. sie müssen entweder stöchiometrisch eingesetzt werden oder aber durch Reduktion eines Cosubstrats regeneriert werden (sog. Cofaktor-Regenerierung). Ein Cosubstrat ist dabei definiert als eine Verbindung, die als Oxidationsmittel enzymatisch reduziert wird, wobei die zur Reduktion erforderlichen Elektronen von NADH bzw. NADPH übertragen werden und somit NAD bzw. NADP regeneriert wird.

Ein chiraler Alkohol mit großer Bedeutung in der synthetischen Chemie ist (S)-2-Butanol.

Allen gängigen Ansätzen zur Herstellung von (S)-2-Butanol ist gemein, dass entweder aufwändig Derivate hergestellt werden müssen zur Enantiomerentrennung (z. B. durch enzymatische kinetische Resolution, EKR oder durch chemische Enantiomerentrennung) oder dass bei der direkten enantioselektiven Reduktion von 2-Butanon zu (S)-2-Butanol die Enantiomerenreinheit von (S)-2-Butanol für technische Anwendungen nicht ausreichend ist (z. B. durch selektive chemische oder enzymatische Reduktion).

Der einfachste Ansatz zur Herstellung von (S)-2-Butanol wäre die direkte enantioselektive Reduktion von 2-Butanon, entweder enzymatisch durch eine S-spezifische CR oder durch katalytische Hydrierung. Hier ergibt sich aber das Problem, dass die Methyl- und Ethylgruppe benachbart zur prochiralen Carbonylgruppe in 2-Butanon sterisch zu wenig unterschiedlich sind, um eine ausreichende Selektivität bei der Reduktion zu gewährleisten.

WO 2005/108593 beschreibt ein Verfahren zur direkten Reduktion von 2-Butanon zu (S)-2-Butanol in einem 2-Phasensystem mittels einer CR und einem Cofaktor. Der Cofaktor wird mittels eines Cosubstrats regeneriert. Zur Cofaktor-Regenerierung wird ein nicht mit Wasser mischbarer sekundärer Alkohol, in hohem molarem Überschuss verwendet. Das Verfahren ist allerdings mit hohen Herstellungskosten verbunden, da der Reaktionsumsatz mit 68 % relativ niedrig ist und zur Cofaktor-Regenerierung ein teurer, nicht wassermischbarer sekundärer Alkohol, bevorzugt 2-Heptanol, notwendig ist und die verwendete CR (CR aus Candida parapsilosis) teuer ist. Der sekundäre Alkohol soll neben seiner Funktion als Cosubstrat wegen seiner Nichtmischbarkeit mit Wasser zur Produktabtrennung während und nach der Reaktion dienen. Da das Produkt (S)-2-Butanol jedoch sehr gut wasserlöslich ist, stellt sich bei einer Extraktion mit einem nicht wassermischbaren sekundären Alkohol ein Verteilungsgleichgewicht für das Produkt ein, was zu weiteren deutlichen Ausbeuteverlusten führt. Insgesamt lässt sich (S)-2-Butanol mit diesem Verfahren zwar in ausreichend hoher optischer Reinheit herstellen, allerdings zu hohen Herstellungskosten.

Von Stampfer et al. (2003), Tetrahedron Asymmetry 14: 275-280 wurde ein Verfahren zur oxidativen Racematspaltung mit einer S-selektiven ADH aus Rhodococcus ruber beschrieben, mit der R-Alkohole mit hohem ee hergestellt werden konnten. Allerdings eignete sich dieses Verfahren nicht zur Herstellung von (R)-2-Butanol aus rac-2-Butanol, so dass dieses Verfahren nicht als Anleitung zur Entwicklung eines analogen Verfahrens zur Herstellung von (S)-2-Butanol herangezogen werden konnte.

JP2004-254549 offenbart eine CR, die bevorzugt (R)-2-Butanol gegenüber (S)-2-Butanol zu 2-Butanon oxidiert. Die relative Aktivität der offenbarten CR für (R)-2-Butanol ist allerdings nur um den Faktor 2 höher als für (S)-2-Butanol (siehe Tabelle 6 in JP2004-254549). Die Selektivität der offenbarten CR ist somit nicht ausreichend, um (S)-2-Butanol in genügend hoher Enantiomerenreinheit herzustellen.

Daher werden üblicherweise optische Reinheiten (ausgedrückt als Enantiomerenüberschuss ee) von weniger als 90 % ee erzielt. So wurde eine käufliche Charge von (S)-2-Butanol (Fa. Aldrich) durch chirale Gaschromatographie (GC) untersucht und ein ee von 82,2 % bestimmt (siehe 1. Beispiel). Bei der selektiven enzymatischen Reduktion von 2-Butanon mit einer S-selektiven CR (Enzym T-ADH, kommerziell von der Fa. Jülich Chiral Solutions GmbH erhältlich) wurde (S)-2-Butanol mit einem maximalen ee von 62,5 % erhalten (siehe 8. Beispiel).

Nach dem Stand der Technik ist es also offensichtlich nur mit hohen Kosten oder großem Aufwand möglich, (S)-2-Butanol in effizienter Weise mit einem ee > 90 % herzustellen.

Aufgabe der Erfindung war es daher, ein Verfahren zur Verfügung zu stellen, welches es erlaubt, (S)-2-Butanol im technischen Maßstab mit einer optischen Reinheit ee von > 90 %, bevorzugt > 93 %, besonders bevorzugt > 97 % und insbesondere bevorzugt von mehr als 99 % bei gleichzeitig hohen Raum-Zeitausbeuten herzustellen.

Diese Aufgabe wird gelöst durch ein Verfahren, bei dem eine Biotransformationszusammensetzung, umfassend als Edukt ein racemisches Gemisch von 2-Butanol (rac-2-Butanol), eine Oxidoreduktase, einen Redox-Cofaktor und ein Cosubstrat zur Reaktion gebracht wird, wobei selektiv (R)-2-Butanol zu 2-Butanon oxidiert wird und (S)-2-Butanol in einer optischen Reinheit ee von > 90 % entsteht.

Das erfindungsgemäße Batch-Verfahren ermöglicht die Herstellung von (S)-2-Butanol in hoher optischer Reinheit von > 90 %, bevorzugt > 93 %, besonders bevorzugt > 97 % und insbesondere bevorzugt von mehr als 99 % bei einer hohen Raum-Zeitausbeute und einem geringen Enzymeinsatz. Wie im 5. Beispiel gezeigt, kann mit dem erfindungsgemäßen Verfahren (S)-2-Butanol innerhalb von 8 - 24 h mit Ausbeuten von 30 g/l und mehr erhalten werden, wobei der Einsatz enzymhaltiger Zellen (ausgedrückt in Trockenbiomasse, siehe 1. Beispiel) nicht mehr als 2 g/l Biotransformationsansatz beträgt.

Als Edukt eignet sich beispielsweise ein kommerziell zu günstigen Preisen erhältliches racemisches Gemisch von (S)-2-Butanol und (R)-2-Butanol (rac-2-Butanol).

Bei der Oxidoreduktase handelt es sich vorzugsweise um eine CR die R-Spezifität besitzt.

Bei dem Redox-Cofaktor handelt es sich um eine Verbindung, die in ihrer oxidierten Form in einer ersten enzymatischen Reaktion Elektronen von einer R-spezifischen CR aufnimmt und in einer zweiten enzymatischen Reaktion auf das Cosubstrat überträgt. Die Elektronen der ersten enzymatischen Reaktion stammen dabei aus der Oxidation von (R)-2-Butanol, enthalten in rac-2-Butanol, durch die R-spezifische CR, mit dem Resultat, dass nach Beendigung der Reaktion (R)-2-Butanol vollständig in 2-Butanon umgewandelt ist und (S)-2-Butanol im Reaktionsgemisch verbleibt. Der Redox-Cofaktor ist vorzugsweise ausgewählt aus Verbindungen der Gruppe NAD, NADP (jeweils oxidierte Form des Cofaktors), NADH oder NADPH (jeweils reduzierte Form des Cofaktors) und deren Salze.

Die Redox-Cofaktoren in ihrer oxidierten Form, NAD bzw. NADP, werden bei der oxidativen Racematspaltung stöchiometrisch verbraucht, d.h. sie müssen entweder stöchiometrisch eingesetzt werden oder aber durch Reduktion eines Cosubstrats regeneriert werden (Cofaktor-Regenerierung). Der stöchiometrische Einsatz von NAD oder NADP ist aufgrund des hohen Preises dieser Verbindungen unwirtschaftlich. Dieser Nachteil kann durch die Cofaktor-Regenerierung umgangen werden. Voraussetzung dafür sind ein billiges Cosubstrat (Oxidationsmittel), welches enzymatisch durch die CR reduziert werden kann. Erst die effiziente und billige Regenerierung des Redox-Cofaktors ermöglicht den technischen Einsatz der oxidativen Racematspaltung.

Das Cosubstrat ist eine Verbindung, die als Oxidationsmittel enzymatisch durch die CR reduziert werden kann, wobei die Elektronen von NADH bzw. NADPH auf das Cosubstrat übertragen werden und somit NAD bzw. NADP regeneriert wird.

Beim Einsatz einer CR aus der Klasse der ADHs ist die Cofaktor-Regenerierung durch Reduktion einer billigen Carbonylverbindung als Cosubstrat möglich. Als Cosubstrat vorzugsweise geeignet ist ein Keton oder ein Diketon und besonders bevorzugt Aceton, 2-Butanon oder 2-Pentanon. Diese Cosubstrate haben die Eigenschaft, dass ihre Reduktion reversibel ist und die gebildeten Alkohole durch die ADH auch wieder oxidiert werden können, so dass sich im Laufe der Reaktion ein Reaktionsgleichgewicht einstellen wird. Mit einem Cosubstrat aus der Klasse der Verbindungen, das durch eine R-spezifische ADH reversibel reduziert wird, wie z. B. Aceton, ist (S)-2-Butanol mit einer optischen Reinheit ee von 93 % herstellbar (siehe 4. Beispiel).

Eine andere Klasse kostengünstiger Cosubstrate sind Verbindungen aus der Klasse der sog. β-Ketoester, und hier beispielhaft als einfachster Vertreter Acetessigsäuremethylester. Diese Verbindungen werden durch ADHs zum entsprechenden β-Hydroxyester reduziert. Bei der entsprechenden Reduktion von Acetessigsäuremethylester entsteht dabei 3-Hydroxy-Buttersäuremethylester.

Cosubstrate aus der Klasse der β-Ketoester haben die Eigenschaft, dass sie durch ADHs irreversibel zu β-Hydroxyestern reduziert werden.

Nun wurde überraschend gefunden, dass die Kombination von ADH und irreversibel reduzierbarem Cosubstrat, ausgewählt aus der Klasse der β-Ketoester, bei der oxidativen Racematspaltung von rac-2-Butanol zu einer höheren Enantiomerenreinheit von S-2-Butanol führt als die Kombination von ADH und reversibel reduzierbarem Cosubstrat.

Besonders bevorzugt zur Cofaktor-Regenerierung ist daher ein Cosubstrat aus der Klasse der β-Ketoester.

Insbesondere bevorzugte Cosubstrate sind Ester der Acetessigsäure, speziell Methyl-, Ethyl-, Isopropyl- und tertiär-Butylester.

Als R-spezifische CRs werden vorzugsweise sekundäre ADHs, z. B. aus Stämmen der Gattung Lactobacillus, wie die ADHs aus Lactobacillus brevis (LB-ADH), Lactobacillus kefir, Lactobacillus parabuchneri, Lactobacillus kandleri oder Lactobacillus minor eingesetzt.

Bevorzugte R-selektive CRs sind die LB-ADH und die ADH aus Lactobacillus kefir.

Besonders bevorzugte R-selektive CR ist die LB-ADH.

Die zur oxidativen Racematspaltung verwendeten ADHs können hergestellt werden, indem man den Mikroorganismus kultiviert, aus dem die betreffende ADH stammt. Dies geschieht jeweils in einer dem Fachmann bekannten Art und Weise. Das auf diese Weise hergestellte ADH Enzym kann dann direkt in den Zellen des Produktionswirts weiterverwendet werden, es kann aber auch nach Aufschluss der Zellen als Proteinextrakt, bzw. nach entsprechender Aufarbeitung durch z. B. Säulenchromatographie als gereinigtes Protein eingesetzt werden.

Die Enzymproduktion der CRs kann mit einem sog. Expressionssystem auch in rekombinanter Form erfolgen. Dazu wird das für die betreffende CR kodierende Gen isoliert und, dem Stand der Technik entsprechend, in einen für die Proteinproduktion geeigneten Expressionsvektor kloniert. Nach Transformation des Expressionsvektors in einen geeigneten Wirtsorganismus wird ein Produktionsstamm isoliert. Mit diesem Produktionsstamm lässt sich die CR in an sich bekannter Weise, z. B. durch Fermentation, produzieren. Das auf diese Weise hergestellte CR Enzym kann dann direkt in den Zellen des Produktionswirts weiterverwendet werden oder aber nach Aufschluss der Zellen als Proteinextrakt, bzw. nach entsprechender Aufarbeitung durch z. B. Säulenchromatographie als gereinigtes Protein.

Dem Fachmann ist bekannt, dass die betreffende CR unter Verwendung eines rekombinanten Expressionssystems mit weit höheren Ausbeuten produziert werden kann als durch Kultivierung des O-riginalstammes, aus dem die CR stammt. Die rekombinante Produktion ermöglicht also eine weit kostengünstigere Herstellung der CR und trägt somit zur Verbesserung der Wirtschaftlichkeit des Verfahrens bei.

Bevorzugt ist die Enzymproduktion der erfindungsgemäßen CRs mit einem Expressionssystem in rekombinanter Form.

Für die Enzymproduktion sind bakterielle und eukaryontische Expressionssysteme geeignet. Wirtsorganismen für die Enzymproduktion sind vorzugsweise ausgewählt aus Escherichia coli, Stämmen der Gattung Bacillus, Hefen wie Pichia pastoris, Hansenula polymorpha oder Saccharomyces cerevisiae sowie Pilzen, wie Aspergillus oder Neurospora, sie sind aber nicht auf die genannten Wirtsorganismen beschränkt.

Zu den bevorzugten Expressionssystemen zählen E. coli, Bacillus, Pichia pastoris, S. cerevisiae, Hansenula polymorpha oder Aspergillus.

Besonders bevorzugte Expressionssysteme für die Produktion des ADH Enzyms sind E. coli, Pichia pastoris und S. cerevisiae.

Insbesonders bevorzugtes Expressionssystem für die Produktion des ADH Enzyms ist E. coli.

Um einen möglichst kosteneffizienten Enzymeinsatz zu erreichen, erfolgt die Enzymproduktion vorzugsweise durch Fermentation, besonders bevorzugt in einem Fed Batch Verfahren.

Vorzugsweise werden die Fermenterzellen dann direkt in dem erfindungsgemäßen Verfahren weiterverwendet, so dass das erfindungsgemäße Verfahren als Ganzzellbiotransformation geführt wird. Es ist jedoch auch möglich, im erfindungsgemäßen Verfahren die isolierten Fermenterzellen oder auch nach Aufschluss der Zellen den so erhaltenen Proteinextrakt, oder nach entsprechender Aufarbeitung durch z. B. Säulenchromatographie das so erhaltene gereinigte Protein im erfindungsgemäßen Verfahren einzusetzen.

Besonders bevorzugt ist die Ganzzellbiotransformation, bei der zuerst die Enzymproduktion in einer rekombinanten Wirtszelle mittels Fermentation erfolgt und die Fermenterzellen anschließend direkt in einer erfindungsgemäßen Biotransformation verwendet werden.

Eine erfindungsgemäße Biotransformationszusammensetzung (sog. Batchansatz) umfasst zwischen 1 % (v/v) und 40 % (v/v) bezogen auf den Gesamtansatz Fermentationsmedium Fermenterzellen enthaltend eine CR mit einem Biomasseanteil von 0,05 - 2 % (w/v) und zwischen 2 %(w/v) und 50 % (w/v) des Gesamtansatzes das Edukt rac-2-Butanol, einen Redox-Cofaktor ausgewählt aus den Verbindungen NAD, NADH, NADP, NADPH und deren Salzen in einer Menge zwischen 10 µM und 200 µM und zwischen 10 %(v/v) und 50 % (v/v) und ein Cosubstrat ausgewählt aus der Gruppe der Verbindungen, die durch eine CR reversibel oder irreversibel reduziert wird.

Vorzugsweise handelt es sich bei dem Cosubstrat um eine der bereits genannten Verbindungen und bei der CR um eine Alkohldehydrogenase.

In einer abgewandelten Form des Verfahrens werden eine oder mehrere der genannten Komponenten der Biotransformationszusammensetzung kontinuierlich oder auch diskontinuierlich zudosiert (sog. Fed Batchansatz).

Bevorzugtes Verfahren ist der Batchansatz.

Bevorzugt enthält die Zusammensetzung, zwischen 2 % (v/v) und 30 % (v/v) Fermentationsmedium enthaltend Fermenterzellen mit einer CR mit einem Biomasseanteil von 0,1 - 1,5 % (w/v). Der Biomasseanteil ist definiert als Trockenbiomasse, die man erhält, wenn die Fermenterzellen, z. B. in einem Trockenschrank bei 105°C, bis zur Gewichtskonstanz getrocknet werden.

Insbesondere bevorzugt enthält die Zusammensetzung, zwischen 3 % (v/v) und 20 % (v/v) Fermentationsmedium enthaltend Fermenterzellen mit einer CR mit einem Biomasseanteil von 0,15 - 1 % (w/v).

Eine erfindungsgemäße Biotransformationszusammensetzung zeichnet sich ferner dadurch aus, dass der Anteil an Edukt zwischen 2 % (w/v) und 50 % (w/v) des Gesamtansatzes beträgt.

Bevorzugt liegt der Anteil an Edukt zwischen 3 % (w/v) und 30 % (w/v) des Gesamtansatzes.

Insbesondere bevorzugt ist eine Zusammensetzung, bei der der Anteil an Edukt zwischen 5 % (w/v) und 20 % (w/v) des Gesamtansatzes beträgt.

Eine erfindungsgemäße Biotransformationszusammensetzung zeichnet sich dadurch aus, dass der Anteil an Cosubstrat zwischen 10 % (v/v) und 50 % (v/v) beträgt.

Bevorzugt ist eine Zusammensetzung, bei der der Anteil an Cosubstrat zwischen 20 % (v/v) und 45 % (v/v) beträgt.

Insbesondere bevorzugt ist eine Zusammensetzung, bei der der Anteil an Cosubstrat zwischen 30 % (v/v) und 40 % (v/v) beträgt.

Eine erfindungsgemäße Biotransformationszusammensetzung umfasst den Redox-Cofaktor ausgewählt aus NAD, NADH, NADP oder NADPH, bzw. deren Salze, in einer Menge zwischen 10 µM und 200 µM.

Bevorzugt ist eine Zusammensetzung, bei der der Anteil an Redox-Cofaktor ausgewählt aus NAD, NADH, NADP oder NADPH, bzw. deren Salze, zwischen 15 µM und 150 µM beträgt.

Insbesondere bevorzugt ist eine Zusammensetzung, bei der der Anteil an Redox-Cofaktor ausgewählt aus NAD, NADH, NADP oder NADPH zwischen 20 µM und 80 µM beträgt.

Das erfindungsgemäße Verfahren wird vorzugsweise bei einer Temperatur von 2°C bis 50°C, bevorzugt von 3°C bis 40°C, insbesondere bevorzugt von 5°C bis 30°C durchgeführt.

Vorzugsweise wird es in einem pH-Bereich von 5 bis 10, bevorzugt von 6 bis 9, insbesondere bevorzugt von 7 bis 8,5 durchgeführt. Vorzugsweise ist der Ansatz zur Konstanthaltung des pH Wertes gepuffert. Vorzugsweise erfolgt eine pH-Kontrolle über eine Titrationsvorrichtung, gekoppelt an ein pH-Messgerät (sog. pH-Stat Methode).

Die Reaktionsdauer des erfindungsgemäßen Verfahrens beträgt vorzugsweise 3 h bis 60 h, besonders bevorzugt 5 h bis 40 h, insbesondere bevorzugt 7 h bis 30 h.

Unter den genannten Bedingungen kann (S)-2-Butanol mit einem ee von mehr als 90 %, bevorzugt mehr als 93 % und besonders bevorzugt von mehr als 97 %, insbesondere bevorzugt von mehr als 99 % hergestellt werden.

Durch Anwendung der genannten, erfindungsgemäßen Biotransformationszusammensetzungen war es somit erstmals möglich, (S)-2-Butanol durch oxidative Racematspaltung aus rac-2-Butanol in kostengünstiger Form herzustellen. Die besonderen Vorteile dieser oxidativen Racematspaltung sind die einfache Verfahrensführung und, dass keine zusätzliche Derivatisierung von (S)-2-Butanol oder (R)-2-Butanol zur Racematspaltung erforderlich ist.

Wenn eine Rückgewinnung des Enzyms nicht beabsichtigt ist, erhält man das (S)-2-Butanol durch direkte Destillation aus dem Reaktionsansatz oder durch Extraktion des Reaktionsansatzes nach dem Reaktionsende gefolgt von einer Destillation.

Ein technisch einfaches und kostengünstiges Verfahren zur direkten Destillation aus dem Reaktionsansatz ist im 7. Beispiel beschrieben.

In einer alternativen Verfahrensvariante wird das (S)-2-Butanol durch Extraktion mit einem in der wässrigen Phase nicht löslichen Lösungsmittel aus dem Reaktionsansatz gewonnen.

Die Extraktion des (S)-2-Butanols erfolgt vorzugsweise mit einem mit Wasser nicht mischbaren, organischen Lösungsmittel. Die Extraktion kann dabei diskontinuierlich (batchweise) oder kontinuierlich erfolgen.

Als organische Lösemittel sind alle mit Wasser nicht mischbaren Lösungsmittel geeignet, die (S)-2-Butanol aus der wässrigen Phase isolieren können.

Bevorzugt werden organische Lösemittel, ausgewählt aus der Gruppe der chlorierten Kohlenwasserstoffe, Ester, Ether, Alkane und Aromaten, verwendet.

Besonders bevorzugt werden Methylenchlorid, Chloroform, Ethylacetat, Methylacetat, Propylacetat, Isopropylacetat, Butylacetat, Tertbutylacetat, Diethylether, Diisopropylether, Dibutylether und Methyl-Tertbutylether (MTBE), Pentan, Hexan, Heptan, Toluol oder deren Mischungen verwendet.

Insbesondere bevorzugte Lösungsmittel sind Methylenchlorid, Chloroform, MTBE und Ethylacetat.

Nach der Abtrennung der organischen Extraktionsphase wird diese vorzugsweise destillativ aufgearbeitet, wobei eine Anreicherung des Reaktionsproduktes erreicht und die teilweise bis vollständige Abtrennung von Nebenprodukten vom Extraktionslösemittel bewirkt wird und dieses erneut zur Extraktion eingesetzt werden kann.

In einer weiteren alternativen Ausführungsform des erfindungsgemäßen Verfahrens wird die CR für eine Wiederverwendung aus dem Reaktionsansatz zurückgewonnen und so die Wirtschaftlichkeit des Verfahrens nochmals erhöht. Neben hohen Raum-Zeitausbeuten bewirkt der wiederholte Enzymeinsatz einen geringen spezifischen Enzymverbrauch pro Mol Produkt und ermöglicht somit eine höhere Kosteneffizienz der erfindungsgemäßen oxidativen Racematspaltung.

In dieser Verfahrensvariante wird das (S)-2-Butanol mit einem in der wässrigen Phase nicht löslichen Lösungsmittel aus dem Reaktionsansatz extrahiert und das Enzym aus der wässrigen Phase gewonnen. Voraussetzung dafür ist, dass das Lösungsmittel nicht zur Inaktivierung des Enzyms führt. Zu solchen Lösungsmitteln zählen z. B. MTBE und Butylacetat.

Nach der Abtrennung der organischen Extraktionsphase wird diese vorzugsweise destillativ aufgearbeitet, wobei eine Anreicherung von (S)-2-Butanol erreicht und die teilweise bis vollständige Abtrennung von Nebenprodukten vom Extraktionslösemittel bewirkt wird und dieses erneut zur Extraktion eingesetzt werden kann.

Bevorzugtes Verfahren zur Isolierung von (S)-2-Butanol aus dem Reaktionsgemisch ist die direkte Destillation.

Durch Aufreinigung der organischen Extraktionslösung enthaltend das Rohprodukt, beispielsweise mittels Feindestillation, erhält man das gewünschte Endprodukt (S)-2-Butanol. Das Endprodukt (S)-2-Butanol zeichnet sich typischerweise durch Ausbeuten > 30 %, bevorzugt > 35 %, insbesondere bevorzugt > 40 %, bezogen jeweils auf die Einsatzmenge von rac-2-Butanol. Das Produkt zeichnet sich ferner durch eine chemische Reinheit > 98 %, bevorzugt > 99 % aus.

Das Verfahrensprodukt zeichnet sich durch einen Enantiomerenüberschuss ee > 90 %, bevorzugt ee > 93 % besonders bevorzugt 97% oder mehr und insbesondere bevorzugt mehr als 99 % aus.

Die folgenden Beispiele dienen zur Beschreibung der Erfindung:

### 1. Beispiel:

### Herstellung von LB-ADH durch Fermentation

Das Enzym LB-ADH, sein Gen und die rekombinante Produktion von LB-ADH in E. coli sind in EP796914 offenbart. Es wurde das in E. coli transformierte und in EP796914 offenbarte Plasmid pADH-1 verwendet. Alternativ kann das Enzym als aus rekombinanten E. coli hergestellter Rohextrakt kommerziell von der Fa. Jülich Chiral Solutions GmbH bezogen werden.

### Fermentation von LB-ADH produzierenden E. coli:

Herstellung eines Inokulums für die Fermentation:
1. Vorkultur von E. coli pADH-1 in LBamp Medium. Die Anzucht erfolgte über Nacht auf einem Orbitalschüttler (Infors) bei 120 rpm und 30°C.
   LBamp Medium enthielt Pepton vegetable (Oxoid) 10 g/l; Hefeextrakt (Oxoid) 5 g/l; NaCl 5 g/l und Ampicillin 0,1 g/l.
2. Vorkultur: 100 ml SM3amp Medium wurden in einem 11 Erlenmeyerkolben mit 1,3 ml Schüttelkultur beimpft. Die Anzucht erfolgte für 16 - 18 h bei 30°C und 120 rpm auf einem Orbitalschüttler (Infors) bis zu einer Zelldichte OD₆₀₀/ml von 7 - 10. 100 ml der Vorkultur wurden zum Beimpfen von 11 Fermentermedium verwendet.
   SM3amp Medium enthielt Pepton vegetable (Oxoid) 5 g/l; Hefeextrakt (Oxoid) 2,5 g/l; NaCl 0,1 g/l; Ammoniumsulfat 5 g/l; KH₂PO₄ 3 g/l; K₂HPO₄ 12 g/l; Glucose 5 g/l; MgSO₄ x 7 H₂O 0,3 g/l; CaCl₂ x 2 H₂O 14,7 mg/l; FeSO₄ x 7 H₂O 2 mg/l; Natriumcitrat x 2 H₂O 1 g/l; Vitamin B1 5 mg/l; Spurenelementemix 1 ml/l und Ampicillin 0,1 g/l.
   Der Spurenelementemix hatte die Zusammensetzung H₃BO₃ 2,5 g/l; COCl₂ x 6 H₂O 0,7 g/l; CuSO₄ x 5 H₂O 0,25 g/l; MnCl₂ x 4 H₂O 1,6 g/l; ZnSO₄ x 7 H₂O 0,3 g/l und Na₂MoO₄ x 2 H₂O 0,15 g/l .
   Die Fermentationen wurden in Biostat CT Fermentern der Fa. Sartorius BBI Systems GmbH durchgeführt. Fermentationsmedium war FM2amp. Die Fermentation erfolgte im sog. Fed-Batch Modus.
   FM2amp Medium enthielt Glucose 20 g/l; Pepton vegetable (Oxoid) 5 g/l; Hefeextrakt (Oxoid) 2,5 g/l; Ammoniumsulfat 5 g/l; NaCl 0,5 g/l; FeSO₄ x 7 H₂O 75 mg/l; Na₃Citrat x 2 H₂O 1 g/l; CaCl₂ x 2 H₂O 14,7 mg/l; MgSO₄ x 7 H₂O 0,3 g/l; KH₂PO₄ 1,5 g/l; Spurenelementemix 10 ml/l; Vitamin B1 5 mg/l und Ampicillin 0,1 g/l. Der pH des FM2amp Mediums wurde vor Beginn der Fermentation auf 7,0 eingestellt.
   1 1 FM2amp wurde mit 100 ml Inokulum beimpft. Fermentationstemperatur war 30°C. pH der Fermentation war 7,0 und wurde mit den Korrekturmitteln 25 % NH₄OH, bzw. 6 N H₃PO₄ konstant gehalten. Die Belüftung erfolgte mit Pressluft bei einem konstanten Durchfluss von 5 slpm (Standard Liter pro Minute). Der Sauerstoffpartialdruck pO₂ wurde auf 50 % Sättigung eingestellt. Die Regulierung des Sauerstoffpartialdruckes erfolgte über die Rührgeschwindigkeit (Rührerdrehzahl 450 - 1.300 rpm). Zur Kontrolle der Schaumbildung wurde Struktol J673 (20 - 25 % v/v in Wasser) verwendet.

Im Verlauf der Fermentation wurde der Glucoseverbrauch durch off-line Glucose-Messung mit einem Glucose-Analysator der Fa. YSI bestimmt. Sobald die Glukosekonzentration der Fermentationsansätze ca. 5 g/l betrug (5 - 6 h nach Inokulation), wurde die Zudosierung einer 60 % w/w Glucose Feedlösung gestartet. Die Flußrate des Feeds wurde so gewählt, dass während der Produktionsphase eine Glukosekonzentration von 1 - 5 g/l eingehalten werden konnte.

Induktion der LB-ADH Produktion erfolgte durch Zugabe von IPTG (Stammlösung 100 mM) in einer Konzentration von 0,4 - 0,8 mM, sobald das Zellwachstum im Fermenter eine OD_{600/}ml von 50 - 60 erreicht hatte. Die gesamte Fermentationsdauer betrug 32 h. Nach Beendigung der Fermentation wurde die Fermenterbrühe in Aliquots zu je 100 ml eingefroren. Durch Trocknung eines Aliquots der Fermenterzellen in einem Trockenschrank bei 105°C zur Gewichtskonstanz wurde die Trockenbiomasse bestimmt. Sie betrug 50 g/l Fermenterbrühe.

### 2.Beispiel:

### Herstellung eines LB-ADH Rohextrakts

2 1 Zellsuspension aus der Fermentation von E. coli pADH-1 (siehe 1. Beispiel) wurden zentrifugiert (15 Min. 8.000 rpm bei 4°C, GS 3-Rotor, Sorvall Zentrifuge). Das Sediment wurde in 500 ml 50 mM Kaliumphosphat, pH 7,0, 1 mM MgCl₂ resuspendiert und durch drei Passagen durch einen Hochdruckhomogenisator (NS1001L Panda 2K der Fa. Niro Soavi) bei 800 bar Druck aufgeschlossen. Das Homogenat wurde zentrifugiert (30 Min. 8.000 rpm bei 4°C, GS 3-Rotor, Sorvall Zentrifuge). Der Überstand ergab einen LB-ADH Rohextrakt von 535 ml Volumen. Die Bestimmung der LB-ADH Aktivität ergab eine Volumenaktivität von 1.300 U/ml, bzw. eine spezifische Aktivität von 108 U/mg Protein im Rohextrakt.

Spektralphotometrische Bestimmung der LB-ADH-Aktivität:
Der Messansatz von 1 ml Volumen zur photometrischen Bestimmung von LB-ADH-Aktivität war zusammengesetzt aus Messpuffer (0,1 M Kaliumphosphat, pH 7,0, 0,1 M NaCl, 1 mM MgCl₂), 3 µl Substrat 4-Cl-Acetessigsäureethylester, 0,2 mM NADPH und LB-ADH-haltigem Zellextrakt. Messtemperatur war 25°C. Die Reaktion wurde gestartet durch Zugabe des LB-ADH Zellextrakts und die Extinktionsabnahme infolge des Verbrauchs von NADPH bei einer Wellenlänge von 340 nm gemessen (Extinktionskoeffizient von NADPH: ε= 0,63 x 10⁴ 1 x Mol⁻¹ x cm⁻¹). Ein Unit LB-ADH-Aktivität ist definiert als der Verbrauch von 1 µmol NADPH/Min. unter Testbedingungen.

Zur Bestimmung der spezifischen Aktivität wurde die Proteinkonzentration der Zellextrakte in an sich bekannter Weise mit dem sog. "BioRad Proteinassay" der Fa. BioRad bestimmt.

### 3. Beispiel:

### Gaschromatographische Analytik

Referenzsubstanzen für (R)- und (S)-2-Butanol sowie Methyl-Ethylketon sind kommerziell erhältlich (Aldrich).

### Chirale GC:

Verwendet wurde ein dem Stand der Technik entsprechender Gaschromatograph 6890N der Fa. Agilent inkl. Flammenionisationsdetektor, der mit einer CP-Chirasil-Dex-CB Säule der Fa. Varian (25 m x 0,25 mm) zur chiralen Trennung ausgerüstet war.

Zur gaschromatographischen Trennung wurde ein Temperaturgradient von 40°C - 170°C mit einer Gradientensteilheit von 20°C/Min. eingestellt. Retentionszeiten unter diesen Bedingungen waren:
Methyl-Ethylketon: 5,5 Min.
(R)-2-Butanol: 14,1 Min.
(S)-2-Butanol: 14,4 Min.

Für die Referenzsubstanzen wurde der Enantiomerenüberschuss ee bestimmt. Für (R)-2-Butanol betrug der ee 86,8 %, für (S)-2-Butanol betrug der ee 82,2 %.

### 4. Beispiel:

### Oxidative Racematspaltung von rac-2-Butanol mit LB-ADH Zellen und Aceton als Cosubstrat

Ansatz 1: Ein Reaktionsansatz war zusammengesetzt aus 5 ml (4 g) rac-2-Butanol, 40 ml Aceton, 1,2 ml LB-ADH Zellen, 50 µM NADP und 53,8 ml KPi-Puffer. Die Zusammensetzung von KPi-Puffer war 0,1 M Kaliumphosphat, pH 8,0, 0,1 M NaCl, 1 mM MgCl₂. Der Reaktionsansatz wurde bei 15°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 1 dargestellt. Bei einer Reaktionstemperatur von 15°C und einem pH von 8,0 betrug der Enantiomerenüberschuss ee des Produktes (S)-2-Butanol 92,6 %.

**Tabelle 1**

| Zeit (h) | Methyl-Ethylketon (%) | (R) -2-Butanol (%) | (S)-2-Butanol (%) | ee (S)-2-Butanol (%) |
|---|---|---|---|---|
| 0 | 0 | 49,9 | 50,1 | 0,2 |
| 0,5 | 10,64 | 37,09 | 47,89 | 12,8 |
| 1 | 18,31 | 30,14 | 48,00 | 22,8 |
| 1,7 | 23,04 | 25,51 | 48,15 | 30,8 |
| 2,4 | 29,13 | 19,65 | 48,01 | 42 |
| 3,6 | 34,24 | 14,49 | 47,73 | 53,4 |
| 4,3 | 38,64 | 11,76 | 49,6 | 61,6 |
| 5,4 | 40,69 | 8,15 | 47,82 | 70,8 |
| 5,9 | 41,36 | 7,2 | 47,52 | 73,6 |
| 23,1 | 51,29 | 1,67 | 43,9 | 92,6 |

Ansatz 2: Ein Reaktionsansatz war zusammengesetzt aus 5 ml (4 g) rac-2-Butanol, 40 ml Aceton, 1,2 ml LB-ADH Zellen, 50 µM NADP und 53,8 ml KPi-Puffer. Die Zusammensetzung von KPi-Puffer war 0,1 M Kaliumphosphat, pH 8,0, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 2 dargestellt. Bei einer Reaktionstemperatur von 5°C und einem pH von 8,0 betrug der Enantiomerenüberschuss ee des Produktes (S)-2-Butanol 93,4 %.

**Tabelle 2**

| Zeit (h) | Methyl-Ethylketon (%) | (R) -2-Butanol (%) | (S)-2-Butanol (%) | ee (S) -2-Butanol (%) |
|---|---|---|---|---|
| 0 | 0 | 49, 8 | 50,2 | 0,4 |
| 0,2 | 16,11 | 34,32 | 48,95 | 17,6 |
| 2 | 24,98 | 25,37 | 49,25 | 32,0 |
| 4 | 37,87 | 11,77 | 47,90 | 60,5 |
| 6 | 42,92 | 6, 60 | 47,76 | 75,7 |
| 24 | 51,94 | 1,44 | 42,39 | 93,4 |

Ansatz 3: Ein Reaktionsansatz war zusammengesetzt aus 5 ml (4 g) rac-2-Butanol, 40 ml Aceton, 1,2 ml LB-ADH Zellen, 50 µM NADP und 53,8 ml KPi-Puffer. Die Zusammensetzung von KPi-Puffer war 0,1 M Kaliumphosphat, pH 9,0, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 3 dargestellt. Bei einer Reaktionstemperatur von 5°C und einem pH von 9,0 betrug der Enantiomerenüberschuss ee des Produktes (S)-2-Butanol betrug 92,8 %.

**Tabelle 3**

| Zeit (h) | Methyl-Ethylketon (%) | (R) -2-Butanol (%) | (S)-2-Butanol (%) | ee (S) -2-Butanol (%) |
|---|---|---|---|---|
| 0 | 0 | 49,7 | 50,3 | 0,6 |
| 0,2 | 14,03 | 36,08 | 49,26 | 15,4 |
| 1,8 | 32,56 | 16,92 | 48,64 | 48,4 |
| 16 | 50,47 | 1,82 | 45,72 | 92,4 |
| 19,9 | 50,22 | 1,71 | 45,00 | 92,6 |
| 21,1 | 50,95 | 1,70 | 45,51 | 92,8 |

### 5. Beispiel:

### Oxidative Racematspaltung von rac-2-Butanol mit LB-ADH Zellen und Acetessigsäuremethylester als Cosubstrat

Ansatz 1: Ein Reaktionsansatz war zusammengesetzt aus 5 ml (4 g) rac-2-Butanol, 20 ml Acetessigsäuremethylester, 1,2 ml LB-ADH Zellen, 50 µM NADP und 73,8 ml KPi-Puffer. Die Zusammensetzung von KPi-Puffer war 0,1 M Kaliumphosphat, pH 8,5, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 4 dargestellt. Bei einer Reaktionstemperatur von 5°C und einem pH von 8,5 betrug der Enantiomerenüberschuss ee des Produktes (S)-2-Butanol 98,1 %.

**Tabelle 4**

| Zeit (h) | Methyl-Ethylketon (%) | (R) -2-Butanol (%) | (S)-2-Butanol (%) | ee (S) -2-Butanol (%) |
|---|---|---|---|---|
| 0 | 0 | 50,1 | 49,9 | -0, 2 |
| 1 | 16,36 | 33,85 | 45,73 | 14,9 |
| 2 | 23,74 | 27,82 | 44,77 | 23,4 |
| 19,3 | 56,04 | 1,00 | 38,87 | 95,0 |
| 23,6 | 56,98 | 0,60 | 38,51 | 96,9 |
| 26,4 | 57,67 | 0,50 | 38,05 | 97,4 |
| 40,5 | 60,64 | 0,42 | 35,76 | 97,7 |
| 48,1 | 63,25 | 0,34 | 35,66 | 98,1 |

Ansatz 2: Ein Reaktionsansatz war zusammengesetzt aus 5 ml (4 g) rac-2-Butanol, 20 ml Acetessigsäuremethylester, 2,4 ml LB-ADH Zellen, 50 µM NADP und 72,6 ml KPi-Puffer. Die Zusammensetzung von KPi-Puffer war 0,1 M Kaliumphosphat, pH 8,5, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 5 dargestellt. Bei einer Reaktionstemperatur von 5°C und einem pH von 8,5 betrug der Enantiomerenüberschuss ee des Produktes (S)-2-Butanol 98,0 %.

**Tabelle 5**

| Zeit (h) | Methyl-Ethylketon (%) | (R)-2-Butanol (%) | (S)-2-Butanol (%) | ee (S)-2-Butanol (%) |
|---|---|---|---|---|
| 0 | 0 | 49,9 | 50,1 | 0,2 |
| 0,2 | 21,43 | 31,14 | 47,43 | 20,7 |
| 1,6 | 32,46 | 21,35 | 46,19 | 36,8 |
| 3,6 | 48,46 | 8,00 | 43,54 | 69,0 |
| 5 | 50,20 | 6,60 | 43,20 | 73,5 |
| 6,5 | 54, 10 | 3,55 | 42,35 | 84,5 |
| 21,7 | 62,75 | 0,46 | 36,60 | 97,5 |
| 25,2 | 63,09 | 0,36 | 35,76 | 98,0 |

Ansatz 3: Ein Reaktionsansatz war zusammengesetzt aus 10 ml (8,1 g) rac-2-Butanol, 40 ml Acetessigsäuremethylester, 4 ml LB-ADH Zellen, 50 µM NADP und 46 ml KPi-Puffer. Die Zusammensetzung von KPi-Puffer war 0,1 M Kaliumphosphat, pH 8,5, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 6 dargestellt. Bei einer Reaktionstemperatur von 5°C und einem pH von 8,5 betrug der Enantiomerenüberschuss ee des Produktes (S)-2-Butanol 98,2 %.

**Tabelle 6**

| Zeit (h) | Methyl-Ethylketon (%) | (R) -2-Butanol (%) | (S)-2-Butanol (%) | ee (S) -2-Butanol (%) |
|---|---|---|---|---|
| 0 | 0 | 49,80 | 50,20 | 0, 4 |
| 0,2 | 18,72 | 34,31 | 46,97 | 15,6 |
| 2,4 | 33,31 | 18,89 | 43,87 | 39,8 |
| 4,2 | 41,91 | 11,32 | 42,40 | 57,9 |
| 5,2 | 46,89 | 7,74 | 41,42 | 68,5 |
| 6 | 49,02 | 6,00 | 40,24 | 74, 0 |
| 7,5 | 50,79 | 3,66 | 39,47 | 83,0 |
| 24,6 | 62,44 | 0,32 | 34,21 | 98,0 |
| 27,4 | 61,88 | 0,30 | 33,79 | 98,2 |

Ansatz 4: Ein Reaktionsansatz war zusammengesetzt aus 10 ml (8,1 g) rac-2-Butanol, 40 ml Acetessigsäuremethylester, 4 ml LB-ADH Zellen, 50 µM NADP und 46 ml KPi-Puffer. Die Zusammensetzung von KPi-Puffer war 0,1 M Kaliumphosphat, pH 8,0, 0,1 M NaCl, 1 mM MgCl₂. Der Reaktionsansatz wurde bei 15°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 7 dargestellt. Bei einer Reaktionstemperatur von 15°C und einem pH von 8,0 betrug der Enantiomerenüberschuss ee des Produktes (S)-2-Butanol nach 8 h Reaktionszeit 98,0 %. Der Gehalt an (S)-2-Butanol im Reaktionsgemisch betrug 30 g/l.

**Tabelle 7**

| Zeit (h) | Methyl-Ethylketon (%) | (R) -2-Butanol (%) | (S)-2-Butanol (%) | ee (S) -2-Butanol (%) |
|---|---|---|---|---|
| 0 | 0 | 49,83 | 50,17 | 0,2 |
| 0,2 | 16,00 | 32,40 | 57,60 | 25,2 |
| 1 | 41,46 | 12,99 | 45,55 | 55,6 |
| 2 | 51,43 | 5,00 | 43,57 | 79,4 |
| 3 | 55,23 | 2,40 | 42,37 | 89,2 |
| 4 | 57,14 | 1,11 | 41,55 | 94,8 |
| 5 | 57,96 | 0,71 | 41,12 | 96.6 |
| 6 | 59,21 | 0,55 | 40,03 | 97,3 |
| 7 | 59,94 | 0,48 | 39,39 | 97,6 |
| 8 | 60,99 | 0,39 | 38,41 | 98,0 |

### 6. Beispiel:

### Oxidative Racematspaltung von rac-2-Butanol mit LB-ADH Enzym und Acetessigsäuremethylester als Cosubstrat

Ein Reaktionsansatz war zusammengesetzt aus 10 ml rac-2-Butanol, 40 ml Acetessigsäuremethylester, 4 ml LB-ADH Enzym (5.200 U, siehe 2. Beispiel), 50 µM NADP und 46 ml KPi-Puffer. Die Zusammensetzung von KPi-Puffer war 0,1 M Kaliumphosphat, pH 8,5, 0,1 M NaCl, 1 mM MgCl₂. Der Reaktionsansatz wurde bei 5°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Der Reaktionsverlauf ist in Tabelle 8 dargestellt. Bei einer Reaktionstemperatur von 5°C und einem pH von 8,5 betrug der Enantiomerenüberschuss ee des Produktes (S)-2-Butanol 97,9 %.

**Tabelle 8**

| Zeit (h) | Methyl-Ethylketon (%) | (R)-2-Butanol (%) | (S)-2-Butanol (%) | ee (S)-2-Butanol (%) |
|---|---|---|---|---|
| 0 | 0 | 49,8 | 50,2 | 0,4 |
| 0,2 | 16,26 | 34,26 | 45,78 | 14,4 |
| 1,2 | 21,79 | 28,99 | 45,30 | 22,0 |
| 2,5 | 33,95 | 19,05 | 43,64 | 39,2 |
| 4,1 | 42,29 | 11,43 | 42,60 | 57,7 |
| 5,4 | 47,99 | 7,05 | 41,53 | 71,0 |
| 20,7 | 57,73 | 0,59 | 38,55 | 97,0 |
| 24,5 | 57,26 | 0,41 | 38,45 | 97,9 |

### 7. Beispiel:

### Herstellung von (S)-2-Butanol im Pilotmaßstab

Der Ansatz (950 1 Endvolumen) war zusammengesetzt aus 380 1 Acetessigsäuremethylester (406,6 kg), 94,6 1 rac-2-Butanol (76,6 kg), 91 10 x Puffer (0,2 M Kaliumphosphat, pH 8,5, 0,2 M NaCl, 10 mM MgCl₂), 457,71 Wasser, 18,9 g NADP Dinatriumsalz (25 µM Endkonzentration) und 10 1 LB-ADH Zellen. Der Reaktionsansatz wurde bei 15°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert. Nach 16,5 h wurde die Reaktion durch Ansäuren mit Phosphorsäure auf pH 3,0, kurzes Aufheizen auf 72°C, gefolgt von einer Neutralisierung mit NaOH auf pH 6,8 gestoppt. Bei einer Sumpftemperatur von 94 - 96°C wurde das Produkt im Gemisch mit 2-Butanon und Wasser abdestilliert. Durch azeotrope Destillation mit MTBE wurde das Wasser aus der Rohfraktion entfernt und 52,3 kg einer 90,5 % (S)-2-Butanol Rohfraktion gewonnen. Durch weitere Destillation dieser Rohfraktion (5 m Stahlkolonne der Fa. Montz, Sumpftemperatur 98 - 115°C) wurden 32,6 kg (S)-2-Butanol (42,6 % Ausbeute bezogen auf die Einsatzmenge rac-2-Butanol) mit einem ee von 98 % und einer Reinheit von 99,2 % gewonnen.

### 8. Beispiel (Vergleichsbeispiel):

### Reduktion von Methyl-Ethylketon zu (S)-2-Butanol mit T-ADH Enzym

Ein Reaktionsansatz war zusammengesetzt aus 5 ml Methyl-Ethylketon, 45 ml Isopropanol, 44 ml KPi-Puffer, pH 7,0, 6 ml T-ADH Enzym (1.200 U T-ADH, von der Firma Jülich Chiral Solutions GmbH bezogen) und 50 µM NADP. Der Reaktionsansatz wurde bei 30°C gerührt. Zu verschiedenen Zeitpunkten wurden 0,1 ml Proben des Reaktionsansatzes genommen, mit 0,9 ml Methylenchlorid extrahiert und durch chirale GC (siehe 3. Beispiel) analysiert.
Der Reaktionsverlauf ist in Tabelle 9 dargestellt. Bei einer Reaktionstemperatur von 30°C und einem pH von 7,0 betrug der Enantiomerenüberschuss ee des Produktes (S)-2-Butanol 8,8 %.

**Tabelle 9**

| Zeit (h) | Methyl-Ethylketon (%) | (R) -2-Butanol (%) | (S)-2-Butanol (%) | ee (S)-2-Butanol (%) |
|---|---|---|---|---|
| 0 | 100 | 0 | 0 | 0 |
| 0,25 | 85,76 | 1,91 | 8,28 | 62,5 |
| 2 | 28,20 | 15,82 | 51,93 | 53,3 |
| 4 | 12,34 | 23,65 | 60,19 | 43,6 |
| 20 | 9,10 | 40,13 | 49,80 | 10,8 |
| 24 | 8,90 | 41,52 | 49,58 | 8,8 |

## Patentansprüche

1. Verfahren, zur Herstellung von (S)-2-Butanol einer optischen Reinheit ee von > 90 %, bei dem eine Biotransformationszusammensetzung, umfassend als Edukt ein racemisches Gemisch von 2-Butanol (rac-2-Butanol), eine Oxidoreduktase, einen Redox-Cofaktor und ein Cosubstrat zur Reaktion gebracht wird, wobei selektiv (R)-2-Butanol zu 2-Butanon oxidiert wird und (S)-2-Butanol in einer optischen Reinheit ee von > 90 % entsteht.

2. Verfahren gemäß Anspruch 1, **dadurch gekennzeichnet, dass** die Oxidoreduktase eine Carbonylreduktase mit R-Spezifität ist.

3. Verfahren gemäß Anspruch 2, **dadurch gekennzeichnet, dass** als R-spezifische Carbonylreduktase eine sekundäre ADH eingesetzt wird.

4. Verfahren gemäß Anspruch 2 oder 3, **dadurch gekennzeichnet, dass** als R-spezifische Carbonylreduktase eine LB-ADH eingesetzt wird.

5. Verfahren gemäß einem der Ansprüche 1 bis 4, **dadurch gekennzeichnet, dass** der Redox-Cofaktor ausgewählt ist aus Verbindungen der Gruppe NAD, NADP, NADH, NADPH und deren Salzen.

6. Verfahren gemäß einem der Ansprüche 1 bis 5, **dadurch gekennzeichnet, dass** das Cosubstrat eine Verbindung ist, die als Oxidationsmittel enzymatisch durch die Oxidoreductase reduziert werden kann, wobei die Elektronen von NADH bzw. NADPH auf das Cosubstrat übertragen werden und somit NAD bzw. NADP regeneriert wird.

7. Verfahren gemäß einem der Ansprüche 1 bis 6, **dadurch gekennzeichnet, dass** die Carbonylreduktase eine Alkohldehydrogenase ist und das Cosubstrat eine Verbindung ist, die durch eine Alkohldehydrogenase reversibel reduziert wird.

8. Verfahren gemäß Anspruch 7, **dadurch gekennzeichnet, dass** das Cosubstrat ausgewählt ist aus der Gruppe der Ketone und Diketone und vorzugsweise Aceton, 2-Butanon oder 2-Pentanon ist.

9. Verfahren gemäß einem der Ansprüche 1 bis 6, **dadurch gekennzeichnet, dass** die Carbonylreduktase eine Alkohldehydrogenase ist und das Cosubstrat eine Verbindung aus der Klasse der β-Ketoester ist.

10. Verfahren gemäß Anspruch 9, **dadurch gekennzeichnet, dass** das Cosubstrat ein Acetessigsäureester ist, vorzugsweise ein Methyl-, Ethyl-, Isopropyl- oder tertiär-Butylester der Acetessigsäure.

11. Biotransformationszusammensetzung umfassend zwischen 1 % (v/v) und 40 % (v/v) bezogen auf den Gesamtansatz Fermentationsmedium Fermenterzellen enthaltend eine CR mit einem Biomasseanteil von 0,05 - 2 % (w/v) und zwischen 2 %(w/v) und 50 % (w/v) des Gesamtansatzes das Edukt rac-2-Butanol, einen Redox-Cofaktor ausgewählt aus den Verbindungen NAD, NADH, NADP, NADPH und deren Salzen in einer Menge zwischen 10 µM und 200 µM und zwischen 10 %(v/v) und 50 % (v/v) ein Cosubstrat ausgewählt aus der Gruppe der Verbindungen, die durch eine CR reversibel oder irreversibel reduziert wird.

12. Verfahren gemäß einem der Ansprüche 1 bis 11, **dadurch gekennzeichnet, dass** es bei einer Temperatur von 2°C bis 50°C, bevorzugt von 3°C bis 40°C, insbesondere bevorzugt von 5°C bis 30°C durchgeführt wird.

13. Verfahren gemäß Anspruch 1 bis 12, **dadurch gekennzeichnet, dass** es in einem pH-Bereich von 5 bis 10, bevorzugt von 6 bis 9, insbesondere bevorzugt von 7 bis 8,5 durchgeführt wird.

14. Verfahren gemäß Anspruch 1 bis 13, **dadurch gekennzeichnet, dass** es über einen Zeitraum von 3 h bis 60 h, besonders bevorzugt 5 h bis 40 h, insbesondere bevorzugt 7 h bis 30 h durchgeführt wird.

15. Verfahren gemäß Anspruch 1 bis 14, **dadurch gekennzeichnet, dass** (S)-2-Butanol aus dem Reaktionsansatz mittels eines mit Wasser nicht mischbaren, organischen Lösungsmittels oder durch Destillation extrahiert wird.
